(19) Europäisches Patentamt
European Patent Office
Office européen des brevets

(11) Veröffentlichungsnummer: **0 090 242**
**A1**

(12) # EUROPÄISCHE PATENTANMELDUNG

(21) Anmeldenummer: **83102481.5**

(22) Anmeldetag: **14.03.83**

(51) Int. Cl.³: **C 07 H 13/12**, A 01 N 47/18

(30) Priorität: **26.03.82 DE 3211302**

(43) Veröffentlichungstag der Anmeldung: **05.10.83**
**Patentblatt 83/40**

(84) Benannte Vertragsstaaten: **AT BE CH DE FR GB IT LI NL**

(71) Anmelder: **BAYER AG, Zentralbereich Patente, Marken und Lizenzen, D-5090 Leverkusen 1, Bayerwerk (DE)**

(72) Erfinder: **Heywang, Gerhard, Dr., Nittumer Weg 4, D-5060 Bergisch-Gladbach 2 (DE)**
Erfinder: **Lockhoff, Oswald, Dr., Bamberger Strasse 3, D-5090 Leverkusen 1 (DE)**
Erfinder: **Kühle, Engelbert, Dr., von-Bodelschwingh-Strasse 42, D-5060 Bergisch-Gladbach 2 (DE)**
Erfinder: **Hammann, Ingeborg, Dr., Lutherstrasse 22, D-4330 Mühlheim/Ruhr (DE)**
Erfinder: **Homeyer, Bernhard, Dr., Obere Strasse 28, D-5090 Leverkusen 3 (DE)**

(54) **Neue N-carboxylierte N-Methylcarbamate, Verfahren zu ihrer Herstellung und ihre Verwendung als Schädlingsbekämpfungsmittel.**

(57) N-Carbonyl-N-methylcarbamidsäure-arylester der Formel

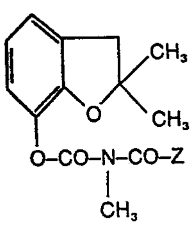

in welcher

Z für den Rest eines gegebenenfalls substituierten Kohlenhydrats steht, bei dem die OH-Gruppen ganz oder zum Teil durch Schutzgruppen substituiert sein können.

Die Verbindungen eignen sich als Schädlingsbekämpfungsmittel, insbesondere als Insektizide.

EP 0 090 242 A1

0090242

BAYER AKTIENGESELLSCHAFT 5090 Leverkusen, Bayerwerk

Zentralbereich
Patente, Marken und Lizenzen

Rt/dr
I b

# Neue N-carboxylierte N-Methylcarbamate, Verfahren zu ihrer Herstellung und ihre Verwendung als Schädlingsbekämpfungsmittel

Die vorliegende Erfindung betrifft neue N-carboxylierte N-Methyl-carbamate, Verfahren zu ihrer Herstellung und ihre Verwendung als Schädlingsbekämpfungsmittel.

Es ist bereits bekannt geworden, daß N-carboxylierte N-Methyl-carbamidsäurearylester (Vergl. DE-OS 2 132 936) und N-Chlorcarbonyl-N-methylcarbamidsäurearylester (Vergl. DE-OS 2 142 496) insektizide Eigenschaften haben. Ihre Wirkung ist jedoch, vor allem bei niedrigen Aufwandmengen nicht immer voll befriedigend.

1. Es wurden gefunden:

Neue N-Carbonyl-N-methylcarbamidsäure-arylester der Formel I

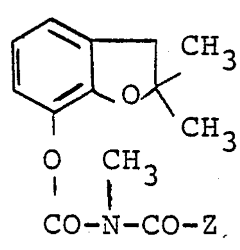

(I)

in welcher

Z für den Rest eines gegebenenfalls substituierten Kohlenhydrats steht, bei dem die OH-Gruppen ganz oder zum Teil durch Schutzgruppen substituiert sein können.

2. Verfahren zur Herstellung von N-Carbonyl-N-methyl-carbamidsäurearylestern der Formel I

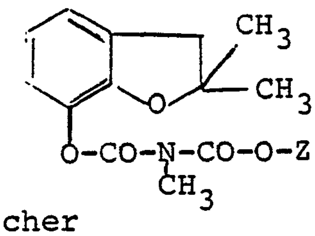

(I)

in welcher

Z für den Rest eines gegebenenfalls substituierten Kohlenhydrats steht, bei dem die OH-Gruppen ganz oder zum Teil durch Schutzgruppen substituiert sein können,

a) indem man 2,3-Dihydro-2,2-dimethyl-7-benzofuranyl-N-chlorcarbonyl-N-methyl-carbamat der Formel II

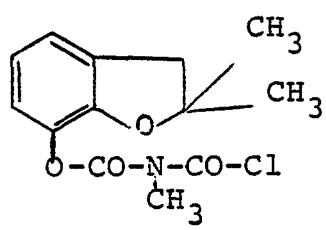

(II)

$a_1$) mit Verbindungen der Formel III

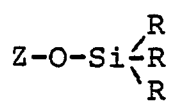

(III)

in welcher

Z die oben angegebene Bedeutung hat und

R für Alkyl steht,

gegebenenfalls in Gegenwart eines Verdünnungsmittels, bei Temperaturen von 0 bis 160°C umsetzt und gegebenenfalls anschließend die Schutzgruppen ganz oder

zum Teil abspaltet, oder

$a_2$) für den Fall, daß die Reaktion an einer primären OH-Gruppe erfolgen soll, mit Verbindungen der Formel IV

$CH_2$-OH
|
Z' (IV)

in welcher

Z' für den Rest eines gegebenenfalls substituierten Kohlenhydrats steht, bei dem die OH-Gruppen ganz oder zum Teil durch Schutzgruppen substituiert sein können, an dem ein $-CH_2-OH$ Rest fehlt,

in Gegenwart einer Base, gegebenenfalls in Gegenwart eines Verdünnungsmittels umsetzt, und gegebenenfalls anschließend die Schutzgruppen ganz oder zum Teil abspaltet,
oder

b) indem man 2,3-Dihydro-2,2-dimethyl-7-benzofuranol der Formel V

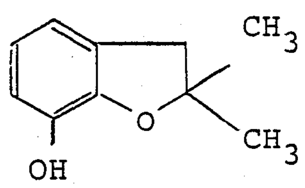

(V)

mit Verbindungen der Formel VI

Cl-CO-N-CO-O-Z"
|
$CH_3$ (VI)

in welcher

Z" für den Rest eines gegebenenfalls substituierten Kohlenhydrats steht, bei dem die OH-Gruppen durch Schutzgruppen substituiert sind

gegebenenfalls in Gegenwart eines Verdünnungsmittels und einer Base umsetzt und gegebenenfalls anschließend die Schutzgruppen ganz oder zum Teil abspaltet.

Die erfindungsgemäßen Verbindungen zeichnen sich durch gute Wirksamkeit als Insektizide, Akarizide und Nematozide aus.

Es ist ausgesprochen überraschend, daß sie bei günstiger Warmblütertoxizität eine höhere Wirkung zeigen als die vom Stand der Technik her bekannten N-carbonylierten N-Methylcarbamate.

Die erfindungsgemäßen Verbindungen sind durch die Formel I allgemein definiert; darin steht Z bevorzugt für den Rest eines Kohlenhydrats mit 3-6 C-Atomen, wobei die Kohlenhydrate mit 5 C-Atomen offenkettig oder als Furanose und die mit 6 C-Atomen offenkettig oder als Pyranose oder als Furanose vorliegen können. Dabei können alle oder einzelne Hydroxygruppen mit gleichen oder verschiedenen Resten substituiert sein. Als Substituenten für die Hydroxylgruppen seien genannt:

Alkyl-, Trialkylsilyl-, Arylalkyl-, Alkoxycarbonyl-, Aryloxycarbonyl-, Alkanoyl-, Aroylreste

Als Substituenten für zwei benachbarte Hydroxylgruppen seien genannt:

Alkyliden- und Benzylidengruppen, die Dioxolringe ausbilden.

Als weitere mögliche Substituenten der Kohlenhydratreste seien genannt: $C_{1-6}$-Alkyl, Halogen, Nitro.

Besonders bevorzugt sind Verbindungen der Formel I, in denen Z für einen Pentose- oder Hexoserest, der als Furanose oder Pyranose vorliegt, steht. Dabei können alle oder einzelne Hydroxylgruppen durch $C_{1-4}$-Alkyl-, Benzyl-, Trimethylsilylreste und benachbarte Hydroxylgruppen durch Isopropyliden- und Benzylidengruppen substituiert sein.

Insbesondere seien folgende Verbindungen der Formel I genannt:

6-O-[N-(2,3-Dihydro-2,2-dimethyl-benzofuranyl-7-oxycarbonyl)-N-methyl-carbamoyl]-1,2-3,4-di-O-isopropyliden-α-D-galactopyranose, Methyl-6-O-[N-(2,3-dihydro-2,2-dimethyl-benzofuranyl-7-oxycarbonyl)-N-methyl-carbamoyl]-2,3,4-tris-O-trimethylsilyl-α-D-glucopyranosid, Benzyl-6-O-[N-(2,3-dihydro-2.2-dimethyl-benzofuranyl-7-oxycarbonyl)-N-methyl-carbamoyl]-2.3.4-tris-O-trimethylsilyl-α-D-glucopyranosid, Methyl-6-O[N-(2,3-dihydro-2,2-dimethylbenzofuranyl-7-oxycarbonyl)-N-methyl-carbamoyl]-2,3,4-tris-O-trimethylsilyl-α-D-mannopyranosid, Benzyl-5-O-[N-(2,3-dihydro-2,2-dimethyl-benzofuranyl-7-oxycarbonyl)-N-methyl-carbamoyl]-2,3-O-isopropyliden-β-D-ribofuranosid, 5-O-[N-(2,2-Dihydro-2,3-dimethyl-benzofuranyl-7-oxycarbonyl)-N-methyl-carbamoyl]-2,3-O-isopropyliden-D-ribofuranose, 6-O-

[N-(2,3-Dihydro-2,2-dimethyl-benzofuranyl-7-oxycarbonyl)-N-methyl-carbamoyl]-D-galactopyranose, Methyl-6-O-[N-(2,3-dihydro-2,2-dimethyl-benzofuranyl-7-oxycarbonyl)-N-methyl-carbamoyl]-α-D-glucopyranosid, Benzyl-6-O-[N-(2,3-dihydro-2,2-dimethyl-benzofuranyl-7-oxycarbonyl)-N-methyl-carbamoyl-α-- D-glucopyranosid, 6-O-[N-(2,3-Dihydro-2,2-dimethyl-benzofuranyl-7-oxycarbonyl)-N-methyl-carbamoyl]-D-glucopyranose, Methyl-6-O-[N-(2,3-dihydro-2,2-dimethylbenzofuranyl-7-oxycarbonyl)-N-methyl-carbamoyl]-α-D-mannopyranosid, 5-O-[N-(2,3-Dihydro-2,2-dimethylbenzofuranyl-7-oxycarbonyl)-N-methyl-carbamoyl]-D-ribofuranose.

Verwendet man im Verfahren 2a$_2$) 2,3-Dihydro-2,2-dimethyl-7-benzofuranyl-N-chlorcarbonyl-N-methyl-carbamat und 1,2,3,4-Di-O-iso-propyliden-α-D-galactopyranose als Ausgansstoffe, so läßt sich der Reaktionsverlauf durch das folgende Reaktionsschema wiedergeben.

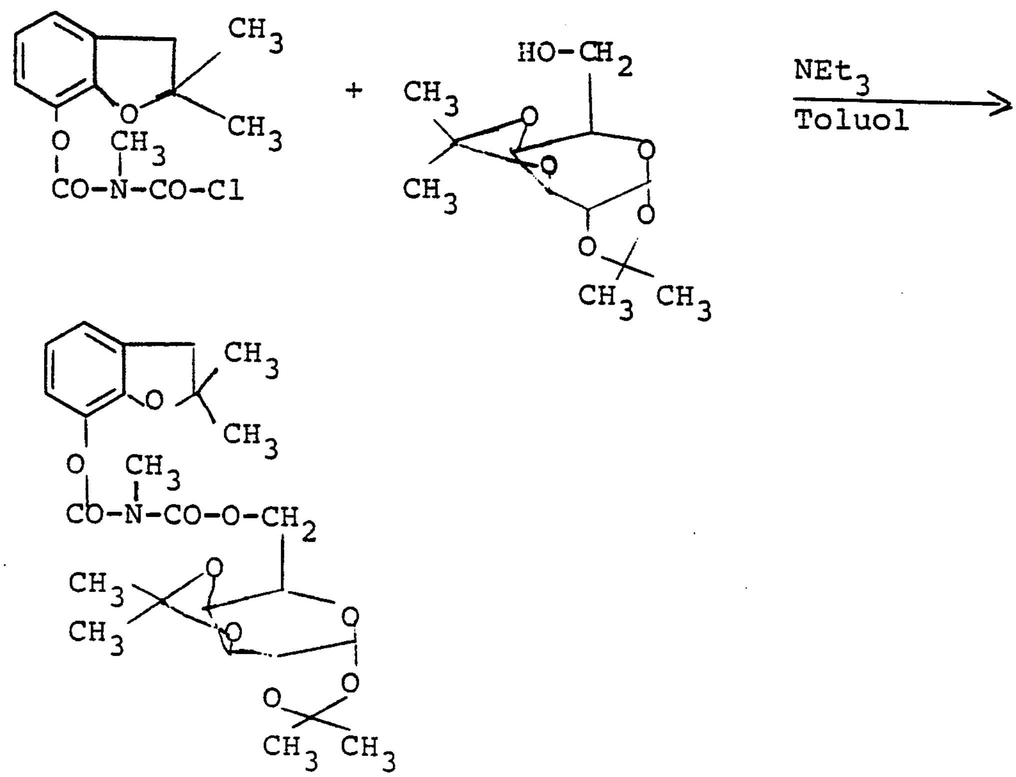

Verwendet man im Verfahren 2b) 2,3-Dihydro-2,2-dimethyl-7-benzofuranol und 6-O-(N-Chlorcarbonyl-N-methylcarbamoyl)-1,2, 3,4-di-O-isopropyliden-α-D-galactopyranose als Ausgangsstoffe, so läßt sich der Reaktionsverlauf durch das folgende Reaktionsschema wiedergeben:

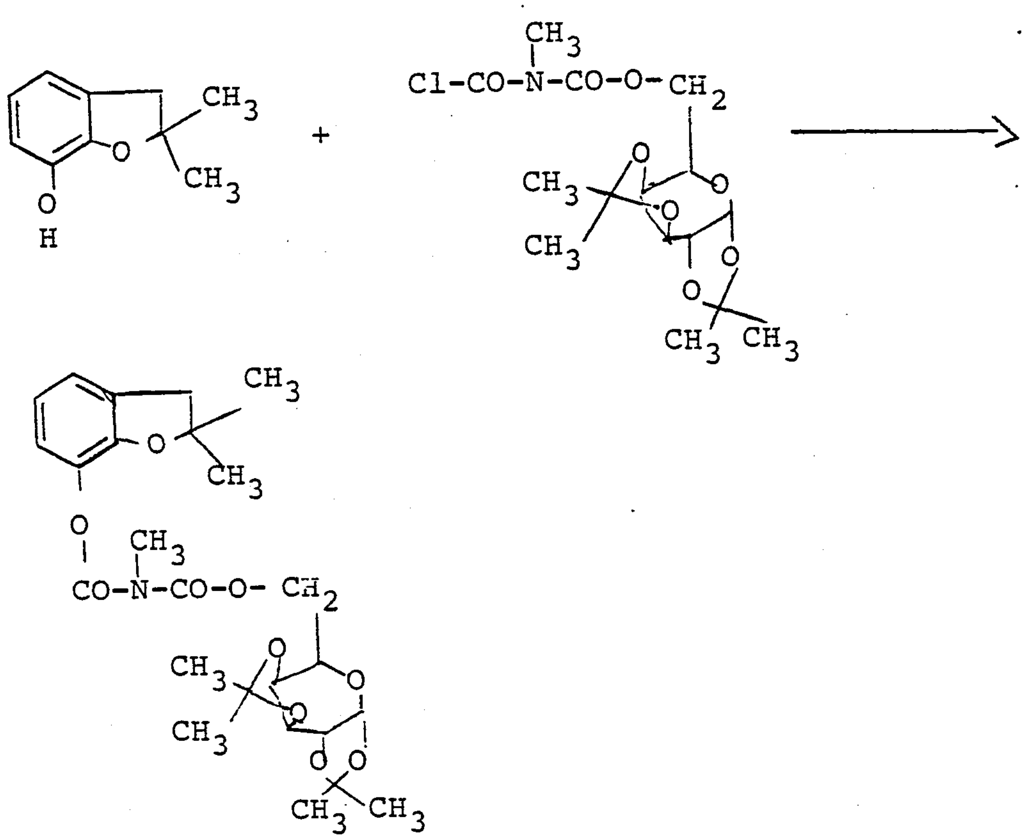

Die bei Verfahren 2 $a_1$ und 2 $a_2$ eingesetzte Verbindung 2,3-Dihydro-2,2-dimethyl-7-benzofuranol-N-chlorcarbonyl-N-methylcarbamat der Formel II ist bekannt.

Die bei Verfahren 2b eingesetzte Verbindung 2,3-Dihydro-2,2-dimethyl-7-benzofuranol der Formel V ist bekannt.

Die bei Verfahren 2b) eingesetzten Verbindungen der Formel VI sind neu. Sie werden erhalten, indem man Verbindungen der Formel IV mit Bischlorcarbonyl-methylamin der Formel VII

$$Cl-CO-\underset{}{\overset{CH_3}{\overset{|}{N}}}-CO-Cl$$

(VII)

in Gegenwart eines säurebindenden Mittels, gegebenenfalls in Gegenwart eines Verdünnungsmittels umsetzt.

Als säurebindendes Mittel setzt man dem Reaktionsgemisch z.B. Natriumcarbonat, Natriumhydrogencarbonat, vorzugsweise eine tertiäre organische Base wie z.B. Triethylamin oder Benzyldimethylamin zu.

Als Verdünnungsmittel für die erfindungsgemäßen Darstellungen eignen sich alle inerten organischen Lösungsmittel. Hierzu gehören Ether wie Diethylether, Dioxan oder Tetrahydrofuran, Kohlenwasserstoffe wie Benzol, Toluol oder Xylol, Chlorkohlenwasserstoffe, wie Dichlormethan oder Chlorbenzol oder Dichlorbenzol, weiterhin Tetrachlorkohlenstoff, Schwefelkohlenstoff, Nitrile, Ester, Ketone sowie Gemische aus diesen Lösungsmitteln.

Die Reaktionstemperatur kann in einem größerem Bereich variiert werden. Im allgemeinen arbeitet man zwischen 0-20°C bis +100°C vorzugsweise zwischen 10-60°C.

Gewöhnlich werden die Reaktionspartner in äquimolaren Mengen eingesetzt. Die Reihenfolge des Zusammengebens der Reaktionspartner ist beliebig, solange das säurebindende Mittel zuletzt zugegeben wird.

0090242

Als Verdünnungsmittel bei den Verfahren $2a_2$) und 2b) eignen sich inerte organische Lösungsmittel. Hierzu gehören Ether wie Diethylether, Dioxan, Tetrahydrofuran, Kohlenwasserstoffe wie Benzol oder Toluol, chlorierte Kohlenwasserstoffe wie Methylenchlorid, Chloroform oder Chlorbenzole, weiterhin Nitrile, Ketone, Ester, sowie Gemische aus diesen Lösungsmitteln.

Als säurebindendes Mittel setzt man dem Reaktionsgemisch Natriumcarbonat, Natriumhydrogencarbonat oder eine tertiäre organische Base, wie z.B. Triethylamin oder Benzyldimethylamin zu.
Die Reaktionstemperatur kann in einem größeren Bereich variiert werden. Im allgemeinen arbeitet man zwischen 0 und 100°C.

Gewöhnlich werden die Reaktionspartner in äquimolaren Mengen eingesetzt, doch auch die Verwendung einer Komponente im Überschuß ist möglich.

Verwendet man im Verfahren $2a_1$) 2,3-Dihydro-2,2-dimethyl-7-benzofuranyl-N-chlorcarbonyl-N-methyl-carbamat und Methyl-2,3,4,6-tetrakis-O-trimethylsilyl-α-D-glucopyranosid als Ausgangsstoffe, so läßt sich der Reaktionsverlauf durch das folgende Reaktionsschema wiedergeben:

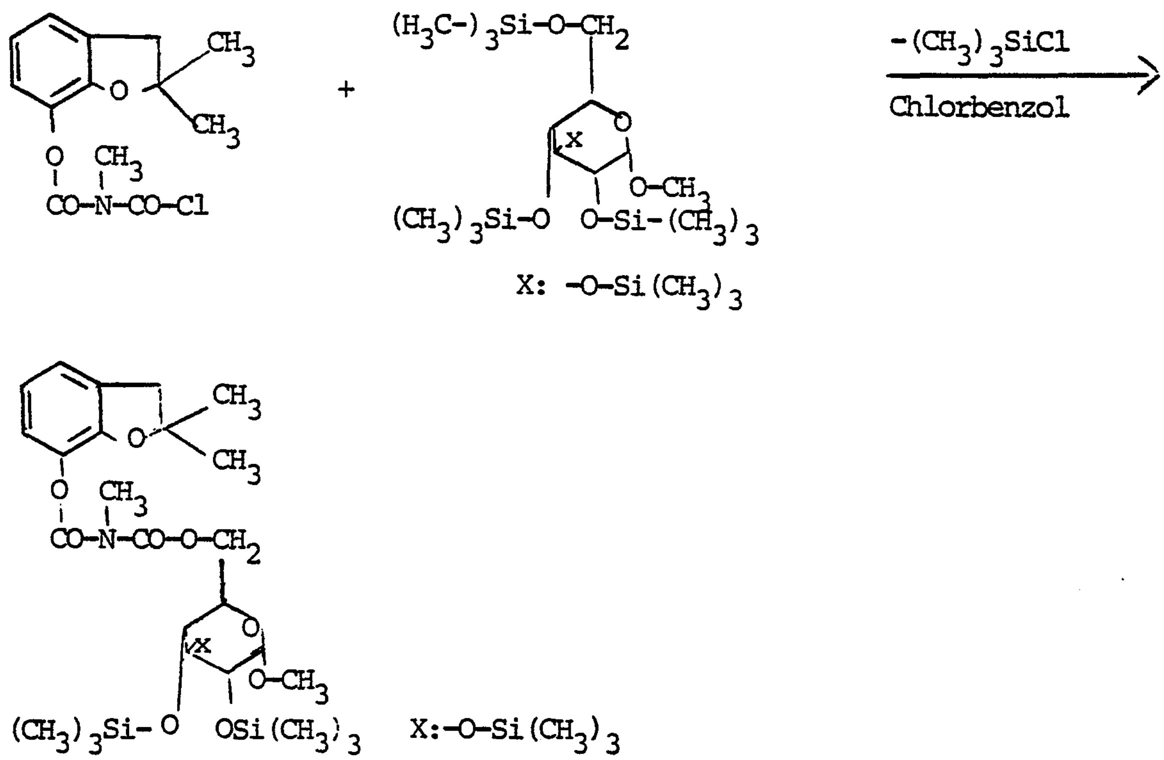


Die bei Verfahren $2a_1$) zusätzlich zur Verbindung der Formel II eingesetzten Verbindungen der Formel III sind zum Teil neu. Sie werden erhalten, indem man Kohlenhydrate, die gegebenenfalls substituiert sein können, in an sich bekannter Weise [Vergleiche D.T. Hurst, A.G. McInnes; Can. J. Chem. 43, 2004 (1965)], mit Trialkylsilylchloriden in Pyridin umsetzt.

Die bei Verfahren $2a_2$) zusätzlich zur Verbindung der Formel II eingesetzten Verbindungen der Formel IV sind zum Teil neu. Sie werden erhalten, in dem man die sekundären OH-Gruppen von Kohlenhydraten in an sich bekannter Weise [Vergleiche O.T. Schmidt, Methods in Carbohydr. Chem. 2, 318 (1963) und E.B. Rauch, D. Läpkin, J. Org. Chem. 27,403 (1962)] derivatisiert z.B. verestert, verethert, oder benachbarte OH-Gruppen acetalisiert. Sie können auch erhalten werden, in dem man trialkylsilylierte Kohlenhydrate bei denen die primäre OH-Gruppe silyliert ist [Vergleiche D.T. Hurst, A.G. McInnes; Can.Chem.J. 43, 2004 (1965)] partiell hydrolysiert.

Als Verdünnungsmittel beim Verfahren $2a_1$) eignen sich inerte organische Lösungsmittel. Hierzu gehören Ether wie Diethylether, Dioxan, Tetrahydrofuran, Kohlenwasserstoffe wie Benzol oder Toluol, chlorierte Kohlenwasserstoffe wie Methylenchlorid, Chloroform oder Chlorbenzole, weiterhin Nitrile, Ketone, Ester, sowie Gemische aus diesen Lösungsmitteln.

Die Reaktionstemperatur kann in einem größeren Bereich variiert werden. Im allgemeinen arbeitet man zwischen 0 und 160 $^{\circ}C$, vorzugsweise zwischen 60 - 140 $^{\circ}C$.

Gewöhnlich werden die Reaktionspartner in äquimolaren Mengen eingesetzt, doch auch die Verwendung einer Komponente im Überschuß ist möglich, bringt jedoch keine wesentlichen Vorteile mit sich.

Die Abspaltung der Schutzgruppen erfolgt nach gängigen Methoden vorzugsweise mit Eisessig-Wassergemischen oder Trifluoressigsäure-Wassergemischen oder durch Hydrierung mit Wasserstoff in Gegenwart eines Katalysators vorzugsweise Palladium auf Aktiv-Kohle.

Die Wirkstoffe eignen sich bei guter Pflanzenverträglichkeit und günstiger Warmblütertoxizität zur Bekämpfung von tierischen Schädlingen, insbesondere Insekten, Spinnentieren und Nematoden, die in der Landwirtschaft, in Forsten, im Vorrats- und Materialschutz sowie auf dem Hygienesektor vorkommen.Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.
Aus der Ordnung der Isoptera z.B. Reticulitermes spp..
Aus der Ordnung der Anoplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.
Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp.,Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.
Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp. Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp.,

Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..
Aus der Ordnung der Arachnida z.B. Scorpio maurus, Latrodectus mactans.
Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Zu den pflanzenparasitären Nematoden gehören Pratylenchus spp., Radopholus similis, Ditylenchus dipsaci, Tylenchulus semipenetrans, Heterodera spp., Meloidogyne spp., Aphelenchoides spp., Longidorus spp., Xiphinema spp., Trichodorus spp..

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstengel; als Emulgier- und/oder schaumerzeugende Mittel kommen in Frage: z.B. nichtionogene und anionische Emulgatoren, wie Polyoxyäthylen-Fettsäure-Ester, Polyoxyäthylen-Fettalkohol-Äther, z.B. Alkylarylpolyglykol-äther, Alkylsulfonate, Alkylsulfate, Acylsulfonate sowie Eiweisshydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 100 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Herstellungsbeispiele

1.) 6-O-[N-(2,3-Dihydro-2,2-dimethyl-benzofuranyl-oxycarbonyl)-N-methyl-carbamoyl]-1,2-3,4-di-O-isopropyliden-α-D-galactopyranose

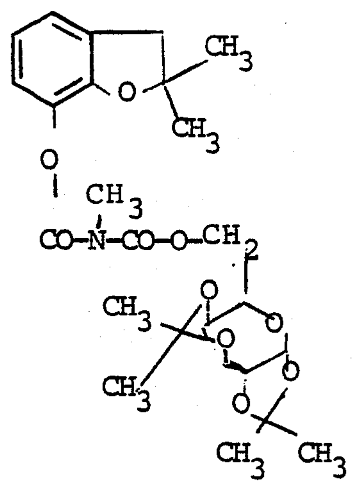

10 g (0,038 mol) 1,2-3,4-Di-O-isopropyliden-α-D-galactopyranose und 10,9 g N-(2,3-Dihydro-2,2-dimethyl-benzofuranyl-7-oxycarbonyl)-N-methylcarbamidsäurechlorid (0,038 mol) werden in 100 ml Toluol vorgelegt und 54 ml (0,038 mol) Triethylamin bei einer Temperatur von 20 °C zugetropft. Nach Beendigung der Zugabe wird das Reaktionsgemisch 2 Stunden auf 40 - 50 °C erwärmt. Der entstandene Niederschlag (Triethylammoniumchlorid) wird abgesaugt, die organische Phase mit Wasser gewaschen, über Magnesiumsulfat getrocknet und schließlich eingeengt. Das zurückbleibende Öl erstarrt bald. Nach Verreiben mit Waschbenzin oder wenig Ethanol hat es einen Schmelzpunkt von 113 °C. Die Ausbeute beträgt 18 g (= 93 % d. Theorie).

1a. 6-O-[N-Chlorcarbonyl-N-methylcarbamoyl]-1,2-3,4-di-O-isopropyliden-α-D-galactopyranose

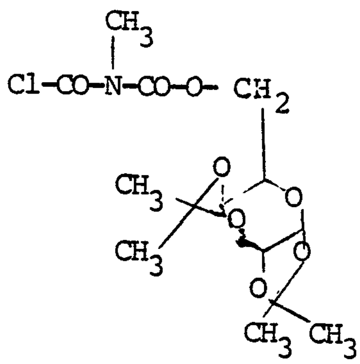

9 g (0,077 mol) 1,2-3,4-Di-O-isopropyliden-α-D-galactopyranose werden in 20 ml Toluol gelöst und bei Raumtemperatur tropfenweise erst mit 12 g (0,077 mol) Bischlorcarbonyl-methylamin und dann mit 10,6 ml (0,077 mol) Triethylamin versetzt. Man läßt nun das Reaktionsgemisch noch 30 min rühren und filtriert den gebildeten Niederschlag ab. Nach dem Abdestillieren des Lösungsmittels bleiben 13 g (46 % d. Theorie) gewünschtes Produkt zurück. ($n_D^{20}$: 1.4839).

1b. 6-O-[N-(2,3-Dihydro-2,2-dimethyl-benzofuranyl-7-oxycarbonyl)-N-methylcarbamoyl]-1,2-3,4-di-O-isopropyliden-α-D-galactopyranose

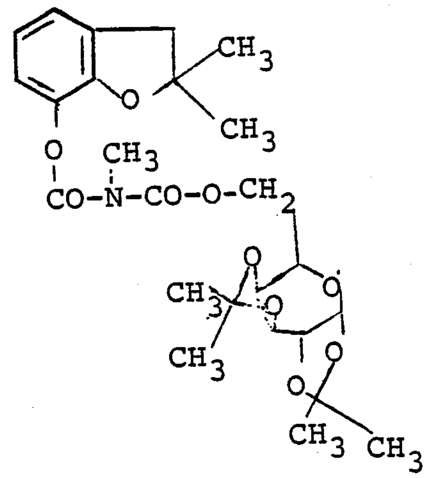

1,6 g (0,01 mol) 2,3-Dihydro-2,2-dimethylbenzofuran-7-ol und 3,8 g (0,01 mol) 6-O-[N-Chlorcarbonyl-N-methylcarbamoyl]-1,2-3,4-di-O-isopropyliden-α-D-galactopyranose werden in 100 ml Toluol vorgelegt und mit 1,4 ml Triethylamin bei einer Temperatur von etwa 20 °C zugetropft. Nach Beendigung der Zugabe wird das Reaktionsgemisch vom Niederschlag abfiltriert, mit Wasser gewaschen, mit Magnesiumsulfat getrocknet und schließlich das Lösungsmittel im Vakuum entfernt. Das rückbleibende Öl erstarrt rasch. 4,9 g (96 % d. Theorie), Schmelzpunkt: 112 °C.

2a. 6-O-[N-(2,3-Dihydro-2,2-dimethyl-benzofuranyl-7-oxycarbonyl)-N-methyl-carbamoyl]-D-galactopyranose

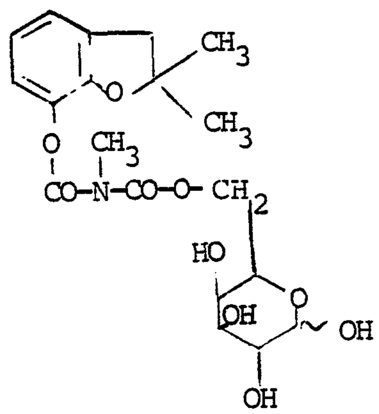

6,8 g (0,013 mol) 6-O-[N-(2,3-Dihydro-2,2-dimethyl-benzofuranyl-7-oxycarbonyl)-N-methylcarbamoyl]-1,2-3,4-di-O-isopropyliden-α-D-galactopyranose werden in 80 ml Eisessig mit 20 ml Wasser 2 h auf 80 - 90 °C erwärmt. Anschließend werden die Lösungsmittel im Vakuum abdestilliert. Der Rückstand wird in Toluol aufgenommen und erneut eingeengt: der letzte Vorgang wird noch zweimal wiederholt. Mit Diisopropylether verrieben kristallisierte der Rückstand. 5,4 g (97 % d. Theorie) Schmp. 60 - 66 °C.

2b. 6-O-[N-(2,3-Dihydro-2,2-dimethyl-benzofuranyl-7-oxycarbonyl)-N-methyl-carbamoyl]-D-galactopyranose

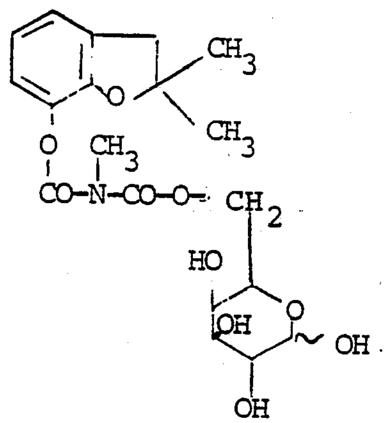

6,8 g (0,013 mol) 6-O-[N-(2,3-Dihydro-2,2-dimethyl-benzofuranyl-7-oxycarbonyl)-N-methylcarbamoyl]-1,2-3,4-di--O-isopropyliden-α-D-galactopyranose werden in 80 ml Trifluoressigsäure mit 20 ml Wasser 1 h gerührt. Anschließend werden die Lösungsmittel im Vakuum abdestilliert. Der Rückstand wird in Toluol aufgenommen und erneut eingeengt: der letzte Vorgang wird noch zweimal wiederholt. Mit Diisopropylether verrieben kristallisierte der Rückstand. 5,0 g (95 % d. Theorie) Schmp. 65 - 66 °C.

3. Benzyl-5-O-[N-(2,3-dihydro-2,2-dimethylbenzofuranyl-7-oxycarbonyl)-N-methyl-carbamoyl]-2,3-O-isopropyliden-β-D-ribofuranosid

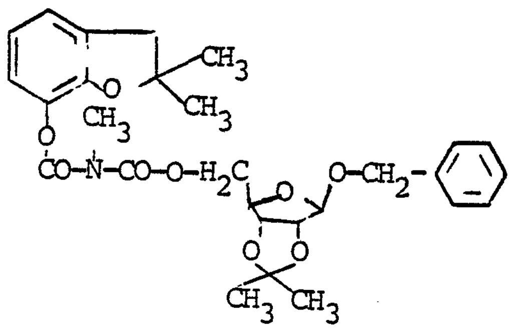

2,8 g (0,01 mol) Benzyl-2,3-O-isopropyliden-β-D-ribofuranosid werden zusammen mit 2,83 g (0,01 mol) 2,3-Dihydro-2,2-dimethyl-7-benzofuranyl-N-methyl-N-chlorcarbonyl-carbamat in 40 ml Toluol vorgelegt und bei Raumtemperatur 1 g Triethylamin zugetropft. Das Reaktionsgemisch wird noch 2 Stunden weitergerührt und anschließend auf Eiswasser gegossen. Die organische Phase wird abgetrennt, über Magnesiumsulfat getrocknet und im Vakuum eingeengt. Der Rückstand kristallisiert und hat einen Schmelzpunkt von 85 °C. Nach Chromatographie über eine Kieselgelsäule mit Toluol-Essigsäureethylester ( 8 : 1) hat die Verbindung einen Schmelzpunkt von 96 - 98 °C.

4. Methyl-6-O-[N-(2,3-dihydro-2,2-dimethyl-benzofuranyl-7-oxycarbonyl)-N-methyl-carbamoyl]-2,3,4-tris-O-trimethylsilyl-α-D-glucopyranosid

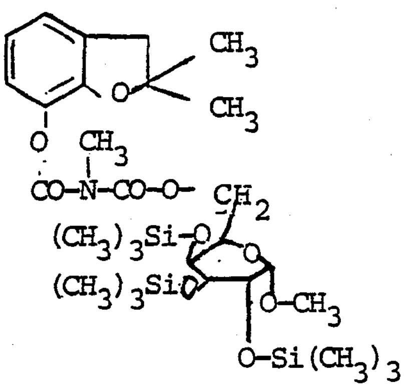

2,83 g (0,01 mol) 2,3-Dihydro-2,2-dimethyl-7-benzofuranyl -N-methyl-N-chlorcarbonyl-carbamat und 5 g (0,01 mol) Methyl-6-O-2,3,4,6-tetrakis-O-trimethylsilyl-α-D-glucopyranosid werden in 20 ml Chlorbenzol 2 Stunden zum Sieden erhitzt und anschließend das Lösungsmittel mit dem abgespaltenen Trimethylchlorsilan abdestilliert. Der Rückstand ist ein zähes Öl (6 g = 91 % d. Theorie, $n_D^{20}$: 1,5012).

 0090242

Beispiel A

Laphygma-Test

| | | |
|---|---|---|
| Lösungsmittel: | 3 Gewichtsteile | Dimethylformamid |
| Emulgator: | 1 Gewichtsteil | Alkylarylpolyglykoläther |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen des Eulenfalters (Laphygma frugiperda) besetzt, solange die Blätter noch feucht sind.

Nach der gewünschten Zeit wird die Abtötung in % bestimmt. Dabei bedeutet 100 %,daß alle Raupen abgetötet wurden; 0 % bedeutet, daß keine Raupen abgetötet wurden.

Bei diesem Test zeigen z.B. die folgenden Verbindungen der Herstellungsbeispiele überlegene Wirksamkeit gegenüber dem Stand der Technik: 1

Beispiel B

Doralis-Test

Lösungsmittel: 3 Gewichtsteile Dimethylformamid
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Bohnentriebe (Vicia faba), die stark von der schwarzen Bohnenlaus (Doralis fabae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, daß alle Blattläuse abgetötet wurden; 0 % bedeutet, daß keine Blattläuse abgetötet wurden.

Bei diesem Test zeigen z.B. die folgenden Verbindungen der Herstellungsbeispiele überlegene Wirksamkeit gegenüber dem Stand der Technik: 1, 2

Beispiel C'

Grenzkonzentrations-Test / Wurzelsystemische Wirkung

Testinsekt: Phaedon cochleariae - Larven
Lösungsmittel: 3 Gewichtsteile Aceton
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (=mg/l) angegeben wird. Man füllt den behandelten Boden in Töpfe und bepflanzt diese mit Kohl (Brassica oleracea). Der Wirkstoff kann so von den Pflanzenwurzeln aus dem Boden aufgenommen und in die Blätter transportiert werden.

Für den Nachweis des wurzelsystemischen Effektes werden nach 7 Tagen ausschließlich die Blätter mit den obengenannten Testtieren besetzt. Nach weiteren 2 Tagen erfolgt die Auswertung durch Zählen oder Schätzen der toten Tiere. Aus den Abtötungszahlen wird die wurzelsystemische Wirkung des Wirkstoffs abgeleitet. Sie ist 100 %, wenn alle Testtiere abgetötet sind und 0 %, wenn noch genau so viele Testinsekten leben wie bei der unbehandelten Kontrolle.

Bei diesem Test zeigen z.B. die folgenden Verbindungen der Herstellungsbeispiele überlegene Wirkung gegenüber dem Stand der Technik: 2

Beispiel D

Grenzkonzentrations-Test / Wurzelsystemische Wirkung

Testinsekt: Myzus persicae

Lösungsmittel: 3 Gewichtsteile Aceton

Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (=mg/l) angegeben wird. Man füllt den behandelten Boden in Töpfe und bepflanzt diese mit Kohl (Brassica oleracea). Der Wirkstoff kann so von den Pflanzenwurzeln aus dem Boden aufgenommen und in die Blätter transportiert werden.

Für den Nachweis des wurzelsystemischen Effektes werden nach 7 Tagen ausschließlich die Blätter mit den obengenannten Testtieren besetzt. Nach weiteren 2 Tagen erfolgt die Auswertung durch Zählen oder Schätzen der toten Tiere. Aus den Abtötungszahlen wird die wurzelsystemische Wirkung des Wirkstoffs abgeleitet. Sie ist 100 %, wenn alle Testtiere abgetötet sind und 0 %, wenn noch genau so viele Testinsekten leben wie bei der unbehandelten Kontrolle.

Bei diesem Test zeigen z.B. die folgenden Verbindungen der Herstellungsbeispiele überlegene Wirkung gegenüber dem Stand der Technik: 1

Beispiel E

Grenzkonzentrations-Test / Bodeninsekten

| | | | |
|---|---|---|---|
| Testinsekt: | | Phorbia antigua-Maden (im Boden) | |
| Lösungsmittel: | 3 | Gewichtsteile | Aceton |
| Emulgator: | 1 | Gewichtsteil | Alkylarylpolyglykoläther |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt. Dabei spielt die Konzentration des Wirkstoffs in der Zubereitung praktisch keine Rolle, entscheidend ist allein die Wirkstoffgewichtsmenge pro Volumeneinheit Boden,welche in ppm ( = mg/l) angegeben wird. Man füllt den Boden in Töpfe und läßt diese bei Raumtemperatur stehen.

Nach 24 Stunden werden die Testtiere in den behandelten Boden gegeben und nach weiteren 2 bis 7 Tagen wird der Wirkungsgrad des Wirkstoffs durch Auszählen der toten und lebenden Testinsekten in % bestimmt. Der Wirkungsgrad ist 100 %, wenn alle Testinsekten abgetötet worden sind, er ist 0 %, wenn noch genau so viele Testinsekten leben wie bei der unbehandelten Kontrolle.

Bei diesem Test zeigen z.B. die folgenden Verbindungen der Herstellungsbeispiele überlegene Wirkung gegenüber dem Stand der Technik: 2

Patentansprüche

1. N-Carbonyl-N-methylcarbamidsäurearylester der Formel I

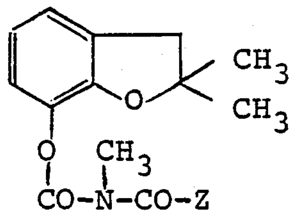

(I)

in welcher

Z für den Rest eines gegebenenfalls substituierten Kohlenhydrats steht, bei dem die OH-Gruppen ganz oder zum Teil durch Schutzgruppen substituiert sein können.

2. Verfahren zur Herstellung von N-Carbonyl-N-methylcarbamidsäurearylester der Formel I

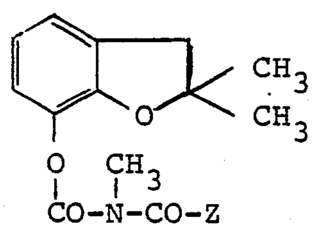

(I)

in welcher

Z für den Rest eines gegebenenfalls substituierten Kohlenhydrats steht, bei dem die OH-Gruppen ganz oder zum Teil durch Schutzgruppen substituiert sein können,

a) indem man 2,3,-Dihydro-2,2-dimethyl-7-benzofuranyl-N-chlorcarbonyl-N-methylcarbamat der Formel II

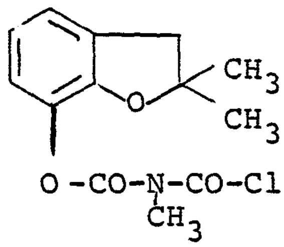

(II)

$a_1$) mit Verbindungen der Formel III

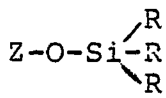

(III)

in welcher

Z die oben angegebene Bedeutung hat und

R für Alkyl steht,

gegebenenfalls in Gegenwart eines Verdünnungsmittels, bei Temperaturen von 0 bis 160°C umsetzt und gegebenenfalls anschließend die Schutzgruppen ganz oder zum Teil abspaltet, oder

$a_2$) für den Fall, daß die Reaktion an einer primären OH-Gruppe erfolgen soll, mit Verbindungen der Formel IV

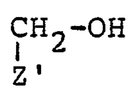

(IV)

in welcher

Z' für den Rest eines gegebenenfalls substituierten Kohlenhydrats steht, bei dem die OH-Gruppen ganz oder zum Teil durch Schutzgruppen substituiert sein können, an dem ein $-CH_2$-OH-Rest fehlt,

 

in Gegenwart einer Base, gegebenenfalls in Gegenwart eines Verdünnungsmittels umsetzt, und gegebenenfalls anschließend die Schutzgruppen ganz oder zum Teil abspaltet,
oder

b) indem man 2,3-Dihydro-2,2-dimethyl-7-benzofuranol der Formel V

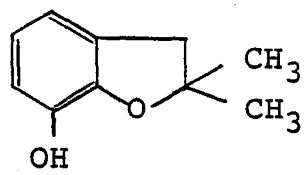

mit Verbindungen der Formel VI

$$\begin{array}{l} Cl-CO-N-CO-O-Z'' \\ \qquad\quad\; CH_3 \end{array} \qquad (VI)$$

in welcher

Z" für den Rest eines gegebenenfalls substituierten Kohlenhydrats steht, bei dem die OH-Gruppen durch Schutzgruppen substituiert sind,

gegebenenfalls in Gegenwart eines Verdünnungsmittels und einer Base umsetzt und gegebenenfalls anschließend die Schutzgruppen ganz oder zum Teil abspaltet.

3. Schädlingsbekämpfungsmittel, gekennzeichnet durch einen Gehalt an mindestens einem N-Carbonyl-N-methylcarbamidsäurearylester der Formel (I).

4. Verwendung von N-Carbonyl-N-methylcarbamidsäurearylester der Formel (I) zur Bekämpfung von Schädlingen.

5. Verfahren zur Bekämpfung von Schädlingen, dadurch gekennzeichnet, daß man N-Carbonyl-N-methylcarbamidsäurearylester der Formel (I) auf Schädlinge und/oder ihren Lebensraum einwirken läßt.

6. Verfahren zur Herstellung von Schädlingsbekämpfungsmitteln, dadurch gekennzeichnet, daß man N-Carbonyl-N-methylcarbamidsäurearylester der Formel (I) mit Streckmitteln und/oder oberflächenaktiven Mitteln vermischt.

7. Verbindungen der Formel VI

$$Cl-CO-\underset{\displaystyle CH_3}{\underset{|}{N}}-CO-O-Z''$$

in welcher

Z" die in Anspruch 2 angegebene Bedeutung hat.

Europäisches Patentamt

# EUROPÄISCHER RECHERCHENBERICHT

0090242
Nummer der Anmeldung

EP 83 10 2481

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| A,D | DE-A-2 132 936 (BAYER) * Seiten 32-34 * | 1,3 | C 07 H 13/02<br>A 01 N 47/18 |
| | ----- | | |
| | | | **RECHERCHIERTE SACHGEBIETE (Int. Cl. 3)** |
| | | | C 07 H 13/00<br>A 01 N 47/00 |

Der vorliegende Recherchenbericht wurde für alle Patentansprüche erstellt.

| Recherchenort | Abschlußdatum der Recherche | Prüfer |
|---|---|---|
| DEN HAAG | 03-06-1983 | VERHULST W. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82